# EUROPEAN PATENT APPLICATION

(11) **EP 4 239 042 A1**
(43) Date of publication of application: **06.09.2023**
(21) Application number: 22382184.4
(22) Date of filing: 01.03.2022
(51) Int. Cl.: C11D 1/62, A61Q 5/12, C07C 209/20, C11D 1/835, C11D 3/00, C11D 3/50, C11D 11/00, C11D 1/74

(54) **SOFTENER FORMULATIONS FOR MAINTAINING OR IMPROVING MOISTURE WICKING**

(71) Applicant: KAO CORPORATION, S.A., 08210 Barberà del Vallès, Barcelona (ES)
(72) Inventor: Pi Boleda, Bernat, 08210 Barberà del Vallès (Barcelona) (ES); Sobrevias Alabau, Jaume, 08210 Barberà del Vallès (Barcelona) (ES); Nogués López, Blanca, 08210 Barberà del Vallès (Barcelona) (ES); Mundó Blanch, Miquel, 08210 Barberà del Vallès (Barcelona) (ES)
(74) Representative: Hoffmann Eitle

(57) **Abstract**

The present invention generally relates to mixtures of cationic surfactants, and compositions comprising such mixtures, which are useful for maintaining or improving moisture wicking; and their use for softening fabrics and/or fibres.

## Description

### FIELD OF THE INVENTION

The present invention generally relates to mixtures of cationic surfactants, and compositions comprising such mixtures, which are useful for maintaining or improving moisture wicking; and their use for softening fabrics and/or fibres.

### BACKGROUND OF THE INVENTION

Quaternary ester ammonium compounds, commonly referred to as "esterquats" (EQ), have found broad use as fabric softener actives due to their high softening performance (e.g., for softening textile fibres and fabrics as well as keratinous fibres, such as hair), their biodegradability, reasonably low aquatic toxicity, and good cosmetic compatibility with the skin.

However, common softener formulations comprising esterquats have the disadvantage of causing a reduction in the tendency of the fabric to absorb water (i.e., wicking), and a corresponding deterioration in the drying properties of fabrics. This is particularly disadvantageous in the area of sportswear, where removal of moisture away from the body is of high importance. In addition, it is preferable that softener formulations are liquid at room temperature, and/or that they are available in highly concentrated, dispersible form to allow for easy dosage and handling.

There have been attempts in the prior art to partially overcome these disadvantages, either by use of high amounts of various additives; or by total replacement of "quat" based fabric softeners. But there is still an unmet demand for formulations having the advantages of esterquats while overcoming the above-described drawbacks.

In view of the above, the present invention aims at the problem of providing esterquat-based softener formulations capable of maintaining or improving moisture wicking of fabrics or fibres. Preferably, the formulations should be liquid and water-dispersible at room temperature; and/or maintain or improve the hydrophilic properties of cotton fabrics or fibres while providing softness; and/or maintain or improve hydrophilic the properties improvement of synthetic fabrics or fibres.

### SUMMARY OF THE INVENTION

As a solution to the above problems, the present invention provides a mixture of cationic surfactants is obtainable from a process comprising the steps: Step I: esterification of a) with b), and Step II: cation formation from the reaction products of Step I, wherein:
a) is a hydroxyl group-containing compound or a mixture of hydroxyl group-containing compounds comprising a.1 and optionally a.2, wherein:
   - a.1 is an alkanolamine or a mixture of alkanolamines of the general formula (I): 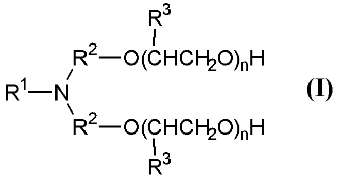 in which R¹ is selected from hydrogen, a C₁-C₆ alkyl group, and the residue 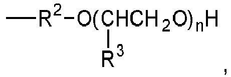 R² is a C₁-C₆ alkylene group, R³ is hydrogen or methyl, n is 0 or an integer from 1 to 20; and
   - a.2 is a polyol, which can be optionally alkoxylated, and is characterized by a MW in the range 60 to 190 g/mol;
b) is a mixture of compounds containing one or more carboxylic groups comprising b.1 and b.2, wherein:
   - b.1 is a monocarboxylic acid or a mixture of monocarboxylic acids of formula (II):

      R⁶-COOH (II)

      in which R⁶ is a linear or branched C₆-C₂₃ alkyl or alkenyl group; or an alkyl ester or glyceride thereof, preferably a linear or branched C₆-C₂₃ alkyl or alkenyl ester; and
   - b.2 is a dicarboxylic acid or a mixture dicarboxylic acids of the general formula (III), or reactive derivative(s) thereof: 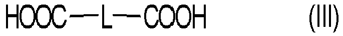 wherein L is a saturated or unsaturated, linear, branched or cyclic group having 1 to 10 carbon atoms, each of which carbon atoms is optionally substituted by a C₁-C₆ saturated or unsaturated group; and is preferably represented by (CH(R⁷))ₘ or by (C₆-C₁₀ arylene) optionally substituted by one or more R⁷, in which each R⁷ is independently a hydrogen, OH or a C₁-C₆ saturated or unsaturated group, m is 0 or an integer from 1 to 10, wherein for m ≥ 2, the chain (CH)ₘ optionally contains one or more double bonds and/or cyclic group(s); wherein a.1), a.2), b.1) and b.2) are introduced in the reaction system of Step I in amounts resulting in the following molar ratios:
   - the molar ratio of monoacid(s)/diacid(s) (b.1/b.2) is 0.10 to 10.0;
   - the molar ratio between organic carboxylic groups and organic hydroxyl groups (COOH/OH) present in the system is 0.30 to 0.80; and
   - the molar ratio between the compound(s) within the definition a.2 and the compound/s under definition a.1 is 0 to 0.5.

The mixture can be used for maintaining or improving moisture wicking properties, e.g., as a component of a softener composition.

The present invention further provides softener compositions comprising the above mixture; as well as concentrated softener compositions comprising such mixture. These compositions can be used for softening while maintaining or improving moisture wicking properties of fabrics and/or fibres.

The present invention further provides use of the mixtures and softener composition(s) for softening fabrics and/or fibres, while maintaining or improving moisture wicking properties.

### DETAILED DESCRIPTION OF THE INVENTION

The present inventors have surprisingly found that particular mixtures of cationic surfactants (esterquats (EQ)) obtainable by reacting a combination of mono- and di-carboxylic acids with alkanolamine(s) and optionally polyol(s), and subsequent cation formation, are capable of providing softening effect while maintaining or improving the moisture wicking capability of fabrics or fibres. These effects can be observed even in the absence of further additives.

Specifically, the inventors have found that the formulations of the invention are capable of keeping or improving the hydrophilic properties (wicking) of untreated cotton fabrics. Unexpected improvement hydrophilic properties on synthetic clothes can also be achieved.

In preferred embodiments, the formulations maintain or improve the hydrophilic properties of cotton fabrics or fibres while providing softness; and/or maintain or improve hydrophilic the properties improvement of synthetic fabrics or fibres.

In preferred embodiments, the formulations (mixtures of cationic surfactants or compositions comprising such mixtures) are liquid and water-dispersible at room temperature.

### Mixtures of cationic surfactants

A mixture of cationic surfactants obtainable from a process as described herein.

In an embodiment of the invention, the molar ratio of monoacid(s)/diacid(s) (b.1/b.2) is preferably 0.2 to 8.0, more preferably 0.30 to 5.0. Without wishing to be bound by theory, it is believed that these ratios can contribute to further improving the wicking properties of fabrics and/or fibres.

In an embodiment of the invention, the molar ratio between organic carboxylic groups and organic hydroxyl groups (COOH/OH) present in the system is preferably 0.40 to 0.70, more preferably 0.50 to 0.70. Without wishing to be bound by theory, it is believed that these ratios can contribute to improving the stability of formulations comprising the mixture of cationic surfactants of the present inventions. Particularly good stability is achieved when the molar ratio between organic carboxylic groups and organic hydroxyl groups (COOH/OH) in the definition of the mixture of cationic surfactant below 0.7.

In an embodiment of the invention, the molar ratio between the compound(s) within the definition a.2 and the compound/s under definition a.1 is 0 or 0.1 to 0.5, preferably 0.1 to 0.5.

In a preferred embodiment of the invention, the molar ratio monoacid/diacid has a value between 0.30 and 5.0; the molar ratio between organic carboxylic groups and organic hydroxyl groups present in the system has a value between 0.50 and 0.70; and he molar ratio between the compound/s under the definition a.2 and the compound/s under definition a.1 is 0 or a value between 0.1 to 0.5. Without wishing to be bound by theory, these particular ratios are believed, individually or in combination, to contribute to wicking properties of fabrics and/or fibres, e.g., to maintain the wicking capability of cotton to above 80%.

Preferably, the cationic surfactant mixtures of the present invention are liquid and dispersible in water at room temperature. More preferably, the cationic surfactant can form, in combination with perfume, homogeneous and stable viscous liquids that can be introduced directly to the washing machine as a tablet or a concentrated product, or can be used to prepare a fabric softener at home using tap water at room temperature. Such combined, concentrated surfactant or surfactant/perfume comprising compositions homogeneous at room temperature, and show good stability upon storage at room temperature, and preferably also under various other conditions (5 °C, 40 °C). In such a preferred embodiment of the invention, the molar ratio of monoacid(s)/diacid(s) (b.1/b.2) is 0.1 to 5.0, preferably 1.0 to 4.0, more preferably 1.5 to 3.5; and/or the molar ratio between organic carboxylic groups and organic hydroxyl groups (COOH/OH) present in the system is 0.30 to 0.70, preferably 0.40 to 0.70, more preferably 0.50 to 0.70; and/or the molar ratio between the compound(s) within the definition a.2 and the compound/s under definition a.1 is 0 to 0.5, preferably 0 or 0.1 to 0.5, more preferably 0.1 to 0.5. Without wishing to be bound by theory, these particular ratios individually or in combination allow the mixtures to be combined with perfume, thereby providing softener compositions that are homogeneous, stable and viscous. Additionally, they can contribute to improving the softening effect and/or the stability of the formulations formed by using the mixtures of cationic surfactants according to the present invention.

Preferably, the cationic surfactant mixtures of the present invention can be directly dispersed or dissolved in water. The thus obtained aqueous softener compositions are preferably optically transparent and stable upon storage under various conditions. Cationic surfactant mixtures according to a preferred embodiment of the present invention can be preferably used to formulate clear fabric softener compositions at 20°C. In such a preferred embodiment of the invention, the molar ratio of monoacid(s)/diacid(s) (b.1/b.2) can be 0.10 to 1.0, preferably 0.20 to 1.0, more preferably 0.30 to 1.0; and/or the molar ratio between organic carboxylic groups and organic hydroxyl groups (COOH/OH) present in the system is 0.30 to 0.70, preferably 0.40 to 0.70, more preferably 0.50 to 0.70; and/or the molar ratio between the compound(s) within the definition a.2 and the compound/s under definition a.1 is 0 or 0.1 to 0.5, preferably 0.1 to 0.5. Without wishing to be bound by theory, each of these particular ratios can contribute to improving the optical transparency. Additionally, handleability (simple handling), and/or softening properties may be improved. The inventors have surprisingly found out that by using the particular ratios of the components, it is possible to obtain cationic surfactant mixtures allowing to prepare clear and stable softener formulations when mixed with water at room temperature, even when using a broader variety of monocarboxylic acid(s) than in the prior art. Particularly good transparency is achieved at a water content lower than 5%, preferably 0-5% w/w, most preferably in the absence or essential absence of water.

Nevertheless, it is not strictly required that the formulations are transparent.

In an embodiment of the invention, the alkanolamine(s) of formula (I) is/are selected from triethanolamine, N-methyldiethanolamine, N-methyldiisopropanolamine and triisopropanolamine, each of which is optionally alkoxylated with ethylene oxide or propylene oxide, and mixtures thereof.

In an embodiment of the invention, in the dicarboxylic acid(s) of formula (III), each L is selected from ethane-1,2-diyl, 1-hydroxyethane-1,2-diyl, *cis*-ethene-1,2-diyl, *trans*-ethene-1,2-diyl, propane-1,2-diyl, propane-1,3-diyl, butane-1,4-diyl, pentane-1,5-diyl, hexane-1,6-diyl, cyclohexane-1,4-diyl, octane-1,8-diyl and 1,4-phenylenyl; preferably butane-1,4-diyl, hexane-1,6-diyl or octane-1,8-diyl.

In an embodiment of the invention, the dicarboxylic acid of formula (III) is selected from succinic, malic, glutaric, adipic, sebacic, pimelic, suberic, maleic and terephthalic acid, acids obtained by thermal oligomerisation of unsaturated fatty acids, and mixtures thereof.

In an embodiment of the invention, the reactive derivative(s) of the dicarboxylic acid(s) of the general formula (III) are one or more selected from halide, anhydride, preferably mixed anhydride with acetic acid or cyclic anhydride.

For example, the monocarboxylic acid(s) of formula (II) are synthetic fatty acids and/or are obtained from fats or oils of natural origin, and are optionally hydrogenated; or are derived from oils of vegetal origin which are optionally hydrogenated.

In an embodiment of the invention, the monocarboxylic acid(s) of formula (II) are selected from those which are obtained from tallow, palm, olive, coconut, sunflower, soya, rapeseed, grape marc and grape, each of which can be hydrogenated, partially hydrogenated, or non-hydrogenated.

In an embodiment of the invention, the iodine value of the carboxylic monoacid(s) of formula (II) is preferably ≥ 5, more preferably ≥ 35, even more preferably ≥ 45, most preferably ≥ 50; and/or preferably ≤ 280, more preferably ≤ 180, even more preferably ≤ 100, most preferably ≤ 80. Preferably, the iodine value of the carboxylic monoacid(s) of formula (II) is 5-280, more preferably 5-180, more preferably 35-180, more preferably 45-100, more preferably 45-80, most preferably 50-80. Without wishing to be bound by theory, using carboxylic monoacid(s) having such iodine values can contribute to even further improving the optical transparency at various temperatures, and/or the storage stability of the aqueous formulations obtained from the mixture of cationic surfactants according to the present invention.

In an embodiment of the invention, the iodine value of the carboxylic monoacid(s) of formula (II) is preferably ≥ 5, more preferably ≥ 40, most preferably ≥ 60; and/or preferably ≤ 280, more preferably ≤ 180, most preferably ≤ 100. Preferably, the iodine value of the carboxylic monoacid(s) of formula (II) is 5-280, more preferably 40-180, most preferably 40-60 or 60-100. Without wishing to be bound by theory, using carboxylic monoacid(s) having such iodine values can contribute to even further improving the wicking properties of fabrics, such as cotton fabrics.

In an embodiment of the invention, the carboxylic monoacid(s) of formula (II) is one or more selected from caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselinic acid, linoleic acid, linolenic acid, eleostearic acid, arachic acid, gadoleic acid, behenic acid and erucic acid, and mixtures thereof which are obtained for example by pressure splitting of natural fats and oils, in the reduction of aldehydes from Roelen's oxosynthesis or dimerization of unsaturated fatty acids, stearic acids, isostearic acid, palmitic acid, myristic acid, lauric acid, capric acid, caprylic acid, 2-ethylhexanoic acid, 2-octyldodecanoic acid, capric acid, oleic acid, linoleic acid, linolenic acid, partially hydrogenated coconut fatty acid, palm fatty acid, partially hydrogenated distilled palm fatty acid, hydrogenated distilled palm fatty acid, palm kernel fatty acid, tallow fatty acid, distilled tallow fatty acid, and rapeseed fatty acid.

In an embodiment of the invention, the compounds corresponding to a.1 and/or a.2 can be from natural origin or from synthetic origin.

In an embodiment of the invention, the polyol a.2 is one or more selected from trimethylolpropane (TMP), glycerine and neopentyl glycol (NPG), each of which can be optionally alkoxylated, preferably ethoxylated; wherein the polyol a.2 is more preferably trimethylolpropane (TMP), or is absent.

Step I is and esterification step of reacting a) with b). In an exemplary embodiment, monoacid b.1 and diacid b.2 are combined with alkanolamine a.1 and optionally the polyol b.2. The obtained mixture is heated. Preferably, the mixture is heated to reflux under atmospheric pressure, e.g., for 1-5, preferably 2-4 hours at 140-200 °C, preferably 160-180°C. Preferably, step I is performed until no more water is distilled off the reaction mixture.

The reaction product obtained from step I is subjected to cation formation in step II. Preferably, an organic solvent is added before step II. Step II can correspond to the formation of the addition salts of the alkanolamine esters obtained from Step I with mineral or organic acids, preferably wherein the mineral or organic acids are one or more selected from hydrochloric, sulphuric, phosphoric, citric and lactic acid. Alternatively, Step II can correspond to the quaternisation of reaction mixtures of Step I with alkylating agent(s), preferably wherein the alkylating agents are one or more selected from methyl chloride, methyl bromide, dimethyl sulphate, diethyl sulphate and dimethyl carbonate. Step II can be performed at room temperature or elevated temperature, e.g., 40-100 °C, preferably 50-90 °C; preferably for 1-5, more preferably 2-4 hours, or until the virtually complete absence of amine value was verified by acid/base assay.

In an embodiment of the invention, the mixture further comprises an organic solvent, preferably an alcohol, more preferably ethanol, n-propanol or isopropanol, butanols, glycol, propane or butanediol, glycerol, diglycol, propyl or butyl diglycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol methyl or ethyl ether, methoxy, ethoxy or butoxy triglycol, 1-butoxyethoxy-2-propanol, 3-methyl-3-methoxybutanol, or propylene glycol t-butyl ether. For instance, such solvent may be added during the preparation step, e.g., during Step I and/or II, preferably before Step II.

In an embodiment of the invention, the content of the organic solvent in the cationic surfactant mixture is 0-30%, preferably 0-20%, more preferably 10-20% by weight.

In an embodiment of the invention, the mixture is essentially water-free.

In an embodiment of the invention, the mixture essentially consists of the reaction products of steps I and II, and optionally an organic solvent. Preferably, the mixture consists of the reaction products of steps I and II and solvent, if any, and unreacted starting materials as well as inevitable impurities from the production process, if any.

### Softener compositions

Further provided is a softener composition, preferably a fabric-softening and/or keratin-based-fibres-softening composition, comprising the cationic surfactant mixture according to the invention. The composition preferably further comprises water.

Preferably, such compositions exhibit can exhibit advantageous storage stability under various conditions, as well as further advantageous properties, such as handleability (simple handling) and/or softening properties.

Such compositions may optionally be clear solutions at RT, and can be stable upon storage RT, or 5°C, or at 40°C, e.g., for at least 2 months. Particularly good stability is achieved when the molar ratio between organic carboxylic groups and organic hydroxyl groups (COOH/OH) in the definition of the mixture of cationic surfactant is 0.7 or less, more preferably below 0.7. In an embodiment of the invention, the composition may be optically transparent at room temperature, and preferably also at temperatures of 26-40 °C, more preferably 20-50 °C, even more preferably 16-50 °C, most preferably 5-60 °C. Particularly good optical transparency if the aqueous formulation is achieved when the molar ratio of monoacid(s)/diacid(s) (b.1/b.2) is 1.0 or below.

In an embodiment of the invention, the composition further comprises a perfume. The perfume the perfume consists of one or more substance(s). The average logP of the perfume substance(s) is from 1 to 6. The weight ratio between the cationic surfactant mixture according to the invention, as defined hereinabove, and the perfume is from 99:1 to 40:60, more preferably from 80:20 to 60:40, even more preferably 80:20 to 70:30. The perfume can be partially encapsulated, preferably the perfume is encapsulated in a biodegradable microcapsule, more preferably the microcapsule is based on chitosan.

In the softener composition, the water content is preferably higher than 50%, more preferably higher than 80%, most preferably higher than 85% w/w. The solid residue is preferably lowerthan 50%, more preferably lower than 25%, even more preferably between 15 and 2%.

In an embodiment of the invention, the softener composition further comprises a non-ionic surfactant. The weight ratio between the cationic surfactant mixture and the non-ionic surfactant is preferably from 100:0 (i.e., no non-ionic surfactant is present) to 70:30. A non-ionic surfactant can advantageously improve solubility of a perfume in the composition, especially when high amounts of perfume are used (e.g., > 1%, optionally up to 3% by weight of the composition, or more). Preferably, when the amount of perfume in the composition is 0.3% by weight or less, no non-ionic surfactant is used; otherwise, a non-ionic surfactant is optionally used. Additionally, the addition of a non-ionic surfactant can even further improve the good stability of the formulation upon storage.

In an embodiment of the invention, the composition further comprises a thickener, e.g., a thickening polymer. The weight ratio the cationic surfactant mixture to the thickener is preferably from 150:1 to 10:5, more preferably from 100:1 to 10:2. A thickener may be added to increase the viscosity of the composition. Suitable thickeners are, e.g., PEG-150 distearate, Hydroxyethyl cellulose, hydroxymethyl cellulose and derivatives thereof, PEG-120 Methyl Glucose Dioleate, PEG-120 Methyl Glucose Trioleate, (and) propanediol, ethoxylated Sorbitan Triisostearate (e.g., PEG-160 Sorbitan Triisostearate, such as Kaopan TW IS-559S from Kao Chemicals Europe, S.L.), and copolymers of acrylamide and dimethyl amino ethyl methacrylate methyl chloride cross- methylene bisacrylamide (such as FLOSOFT 222 manufactured by SNF).

In an embodiment of the invention, the composition comprises: (i) the mixture of cationic surfactants as described hereinabove, and (ii) optionally one or more non-ionic surfactants, wherein the sum of components (i) and (ii) is from 2% to 50% by weight, and wherein:
- the content of component (i) is from 1% to 50%, preferably from 5 to 15% by weight, relative to the weight of the composition; and
- the content of component (ii) is from 0% to 10%, preferably from 0 to 7%; more preferably from 0 to 5%, even more preferably 0 to 2%, most preferably 0 to 0.05% by weight, relative to the weight of the composition.

Preferred embodiments of compositions according to the invention comprise, preferably essentially consist, more preferably consist of: esterquats (EQ) and water; or EQ, water and thickener; or EQ, water and up to 3.0% by weight of perfume; or EQ, water, non-ionic surfactant(s) and perfume; or EQ, water, thickener, and perfume; or EQ, organic solvent, such as ethanol or glycerine, and perfume.

Preferred embodiments of water-free compositions according to the invention comprise, preferably essentially consist, more preferably consist of: esterquats (EQ); or EQ and thickener; or EQ and perfume; or EQ, non-ionic surfactant(s) and perfume; or EQ, thickener, and perfume; or EQ, organic solvent, such as ethanol or glycerine, and perfume.

The present invention further provides a concentrated softener composition comprising the cationic surfactant mixture as described hereinabove, and having a water content lower than 50%, preferably lower than 30%, preferably lower than 5%. Such composition is preferably liquid and transparent at temperature from 26-40 °C, preferably 20-60 °C, more preferably 16-60 °C, most preferably 0-80 °C.

In an embodiment of the invention, the concentrated softener composition further comprises:
A) a perfume, preferably wherein:
   (i) the perfume consists of one or more substance(s); and/or
   (ii) the average logP of the perfume substance(s) is from 1 to 6; and/or
   (iii) the perfume is an encapsulated perfume, preferably the perfume is encapsulated in a biodegradable microcapsule, more preferably the microcapsule is based on chitosan; and/or
B) a non-ionic surfactant,
   which is preferably characterised in that the weight ratio of the described components are values according to the following ratios:
   P:C is a value from 0:100 to 60:40 NI:C is from 0:100 to 30:70
   - P: corresponds to the perfume, optionally microencapsulated perfume;
   - C: corresponds to the cationic surfactant mixture;
   - NI: corresponds to the non-ionic surfactant.

### Compositions suitable for preparing a unit dose softener

The present invention further provides a concentrated composition suitable for preparing a unit dose softener contained in a water-soluble pouch, wherein the composition comprises a mixture of cationic surfactants as described hereinabove, and is liquid and transparent at a temperature from 26-60 °C, preferably 20-60 °C, more preferably 16-80 °C, most preferably 0-80 °C. The concentrated composition preferably has a water content lower than 10%, preferably lower than 5%, preferably lower than 1%, preferably lower than 0.1%, most preferably 0%.

In such concentrated compositions, preferably cationic surfactant mixtures are used wherein the molar ratio of monoacid(s)/diacid(s) (b.1/b.2) is 0.1 to 5.0, preferably 1.0 to 4.0, more preferably 1.5 to 3.5; and the wherein the molar ratio between organic carboxylic groups and organic hydroxyl groups (COOH/OH) present in the system is 0.30 to 0.70, preferably 0.40 to 0.70, more preferably 0.50 to 0.70.

Such compositions may contain a perfume and/or a non-ionic surfactant. The perfume preferably consists of one or more substance(s). The average logP of the perfume substance(s) is from 1 to 6.

Particularly good perfume compatibility is achieved when the ratio of monoacid(s)/diacid(s) (b.1/b.2) 5.0 or below. Particularly good stability of the compositions comprising the mixture of cationic surfactants and perfume is achieved when the molar ratio between organic carboxylic groups and organic hydroxyl groups (COOH/OH) in the definition of the mixture of cationic surfactants is 0.7 or below.

Preferably, the weight ratio of the described components are values according to the following ratios:
P:C is a value from 0:100 to 60:40
NI:C is 0 or a value from 0:100 to 50:50
   - P: corresponds to the weight content of perfume,
   - C: corresponds to the weight content of cationic surfactant mixture according to claims 1 to 4,
   - NI: corresponds to the weight content of non-ionic surfactant.

Preferably, such concentrated compositions essentially consist, or consist of, the mixture of cationic surfactants as described hereinabove, perfume, optionally non-ionic surfactant, and optionally an organic solvent.

### Unit dose softeners

The present invention further provides a fabric conditioner unit dose (i.e., a unit dose softener) comprising: a water-soluble pouch acting as container of a liquid composition; and the concentrated composition suitable for preparing a unit dose, as described hereinabove, contained within or by the pouch. Preferably, the unit dose is a capsule or a tablet. Preferably, the water-soluble pouch is formed of or comprises polyvinyl alcohol.

### Concentrated compositions suitable for being dispersed in cold water by consumer at home

The present invention further provides a concentrated softener composition comprising the cationic surfactant mixture as described hereinabove, and having a water content lower than 50%, preferably lower than 30%, preferably lower than 5%.

In a preferred embodiment, the invention provides a concentrated softener composition suitable for preparing a domestic softener formulation by dilution. Such compositions are preferably liquid at temperature from 26-60 °C, preferably 20-60 °C, more preferably 16-80 °C, most preferably 0-80 °C; comprises the cationic surfactant mixture as described hereinabove, and has a water content lower than 50%, preferably lower than 30%, preferably lower than 5%.

In an embodiment of the invention, such concentrated softener composition further comprises:
A) a perfume, preferably wherein:
   (i) the perfume consists of one or more substance(s); and/or
   (ii) the average logP of the perfume substance(s) is from 1 to 6; and/or
   (iii) the perfume is an encapsulated perfume, preferably the perfume is encapsulated in a biodegradable microcapsule, more preferably the microcapsule is based on chitosan; and/or
B) a non-ionic surfactant,
   and is preferably characterised in that the weight ratio of the described components are values according to the following ratios:
   P:C is a value from 0:100 to 60:40 NI:C is from 0:100 to 30:70
   - P: corresponds to the perfume, optionally microencapsulated perfume;
   - C: corresponds to the cationic surfactant mixture;
   - NI: corresponds to the non-ionic surfactant.

Particularly good stability of the compositions comprising the mixture of cationic surfactants and perfume is achieved when the molar ratio between organic carboxylic groups and organic hydroxyl groups (COOH/OH) in the definition of the mixture of cationic surfactants is below 0.7.

As used herein, the non-ionic surfactant(s) can be selected from fatty acids, linear or branched, alkoxylated or non-alkoxylated esters of fatty acids, especially those containing from 8 to 18 carbon atoms, alkoxylated or non-alkoxylated Guerbet alcohols, optionally alkoxylated glycerol and polyglycerol esters, xylitol esters, alkoxylated or non-alkoxylated sorbitan esters, esters of sugars, such as glucose, fructose, galactose, mannose, xylose, arabinose, ribose, 2-deoxyribose and sucrose, C₈₋₁₈ fatty alcohols, alkyl polyglucosides, non-ionic surfactants with amide groups derived from amines, such as glucamine, and the derivatives of methylethanolamine, diethanolamine, isopropanolamine and monoethanolamine, with linear or branched fatty acids, especially those containing from 8 to 18 carbon atoms, waxes, such as paraffins, microcrystalline waxes derived from petroleum, and synthetic waxes, and pentaerythritol esters, especially having a tallow, hydrogenated tallow, palm, behenic or oleic chain, preferably non-ionic surfactants are selected from glycerine esters that are ethoxylated, sorbitan monoesters and pentaerythritol esters, especially those having a tallow, hydrogenated tallow, palm, behenic or oleic chain.

Suitable non-ionic surfactants are Glycereth-6 Cocoate (e.g., Levenol F-200), Glycereth-17 Cocoate (e.g., Levenol C-201), ethoxylated C₁₃₋₁₅ alcohol (e.g., with 7EO, such as Findet 1315/19), Findet 1618/35; particularly suitable is ethoxylated hydrogenated castor oil (e.g., with 40 EO, such as Findet ARH-52).

The softener composition or the concentrated softener composition may have a viscosity at 20 °C of 200-50,000 cps, as measured on a Brookfield LVT viscometer with spindle 2 at 60 rpm or with spindle 4 at 12 rpm. Preferably, such compositions are non-newtonian and have a viscosity of 200 to 5,000 mPas as measured on a Brookfield LVT viscometer with spindle 4 at 12 rpm, optionally 200 to 800 mPas; or are newtonian and have a viscosity of 200 to 800 mPas as measured on a Brookfield LVT viscometer with spindle 2 at 30 rpm.

As used herein, the non-ionic surfactant(s) can be selected from fatty acids, linear or branched, alkoxylated or non-alkoxylated esters of fatty acids, especially those containing from 8 to 18 carbon atoms, alkoxylated or non-alkoxylated Guerbet alcohols, optionally alkoxylated glycerol and polyglycerol esters, xylitol esters, alkoxylated or non-alkoxylated sorbitan esters, esters of sugars, such as glucose, fructose, galactose, mannose, xylose, arabinose, ribose, 2-deoxyribose and sucrose, C₈₋₁₈ fatty alcohols, alkyl polyglucosides, non-ionic surfactants with amide groups derived from amines, such as glucamine, and the derivatives of methylethanolamine, diethanolamine, isopropanolamine and monoethanolamine, with linear or branched fatty acids, especially those containing from 8 to 18 carbon atoms, waxes, such as paraffins, microcrystalline waxes derived from petroleum, and synthetic waxes, and pentaerythritol esters, especially having a tallow, hydrogenated tallow, palm, behenic or oleic chain, preferably non-ionic surfactants are selected from glycerine esters that are ethoxylated, sorbitan monoesters and pentaerythritol esters, especially those having a tallow, hydrogenated tallow, palm, behenic or oleic chain.

Suitable non-ionic surfactants are Glycereth-6 Cocoate (e.g., Levenol F-200), Glycereth-17 Cocoate (e.g., Levenol C-201), ethoxylated C₁₃₋₁₅ alcohol (e.g., with 7 EO, such as Findet 1315/19), ethoxylated C₁₆₋₁₈ alcohol (e.g. with 23 EO, Findet 1618/35); particularly suitable is ethoxylated hydrogenated castor oil (e.g., with 40 EO, such as Findet ARH-52).

Nevertheless, in any of the compositions described herein, addition of non-ionic surfactants, thickeners and organic solvents is optional, and not required for achieving the advantageous properties of the compositions, such as perfume compatibility, dispersibility, viscosity and/or stability. That is, non-ionic surfactants, thickeners and/or organic solvents may be absent.

The compositions described above may have a viscosity at 20 °C of 200-50,000 cps, as measured on a Brookfield LVT viscometer with spindle 2 at 60 rpm or with spindle 4 at 12 rpm. Preferably, such compositions are non-newtonian and have a viscosity of 200 to 5,000 mPas as measured on a Brookfield LVT viscometer with spindle 4 at 12 rpm, optionally 200 to 800 mPas; or are newtonian and have a viscosity of 200 to 800 mPas as measured on a Brookfield LVT viscometer with spindle 2 at 30 rpm.

For example, concentrated EQ and their mixtures with NI, perfume and/or glycols can have a viscosity values between 100 and 50000 mPa·s. Diluted formulations (from 2% active to 50% active) can have viscosity values between 2 and 500 mPa·s.

### Uses

The present invention provides use of the mixtures of cationic surfactants, the compositions and/or the unit doses as described herein for maintaining or improving moisture wicking properties of fabrics or fibres. Specifically, these can be used for maintaining or improving hydrophilic properties of cotton fabrics or fibres while providing softness. Additionally or alternatively, these can be used for maintaining or improving hydrophilic properties of synthetic fabrics or fibres.

The mixtures, compositions and/or unit doses of the preceding aspects for softening fabrics and/or fibres, such as keratin-based-fibres, comprising a step of contacting the mixture, composition or unit dose with the fabrics and/or fibres.

For example, the use may comprise dispersing the composition in water, preferably tap water; or introducing the unit dose or the composition directly into a washing machine. In such embodiments, wherein the molar ratio of monoacid(s)/diacid(s) (b.1/b.2) is preferably 0.10 to 5.0, preferably 1.0 to 4.0, more preferably 1.5 to 3.5; and/or the molar ratio between organic carboxylic groups and organic hydroxyl groups (COOH/OH) present in the system is 0.30 to 0.70; preferably 0.40 to 0.70, more preferably 0.50 to 0.70.

In preferred embodiments, the use may also comprise preparing an optically transparent composition, e.g., by comprising combining the cationic surfactant mixture or a concentrated composition with water. In such embodiments, wherein the molar ratio of monoacid(s)/diacid(s) (b.1/b.2) is preferably 0.10 to 1.0, more preferably 0.20 to 1.0, most preferably 0.30 to 1.0.

The present invention also provides the following aspects.
1. A mixture of cationic surfactants obtainable from a process comprising the steps:
   Step I: esterification of a) with b), and
   Step II: cation formation from the reaction products of Step I,
   wherein:
      a) is a hydroxyl group-containing compound or a mixture of hydroxyl group-containing compounds comprising a.1 and optionally a.2, wherein:
         - a.1 is an alkanolamine or a mixture of alkanolamines of the general formula (I): 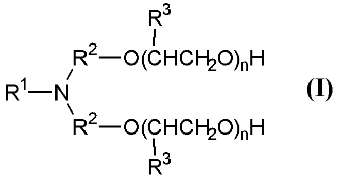 in which R¹ is selected from hydrogen, a C₁-C₆ alkyl group, and the residue 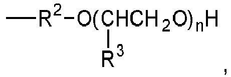 R² is a C₁-C₆ alkylene group, R³ is hydrogen or methyl, n is 0 or an integer from 1 to 20; and
         - a.2 is a polyol, which can be optionally alkoxylated, and is characterized by a MW in the range 60 to 190 g/mol;
      b) is a mixture of compounds containing one or more carboxylic groups comprising b.1 and b.2, wherein:
         - b.1 is a monocarboxylic acid or a mixture of monocarboxylic acids of formula (II):

            R⁶-COOH (II)

            in which R⁶ is a linear or branched C₆-C₂₃ alkyl or alkenyl group; or an alkyl ester or glyceride thereof, preferably a linear or branched C₆-C₂₃ alkyl or alkenyl ester; and
         - b.2 is a dicarboxylic acid or a mixture dicarboxylic acids of the general formula (III), or reactive derivative(s) thereof: 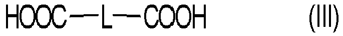 wherein L is a saturated or unsaturated, linear, branched or cyclic group having 1 to 10 carbon atoms, each of which carbon atoms is optionally substituted by a C₁-C₆ saturated or unsaturated group; and is preferably represented by (CH(R⁷))ₘ or by (C₆-C₁₀ arylene)
            optionally substituted by one or more R⁷, in which each R⁷ is independently a hydrogen, OH or a C₁-C₆ saturated or unsaturated group, m is 0 or an integer from 1 to 10, wherein for m ≥ 2, the chain (CH)ₘ optionally contains one or more double bonds and/or cyclic group(s);
         wherein a.1), a.2), b.1) and b.2) are introduced in the reaction system of Step I in amounts resulting in the
      following molar ratios:
      - the molar ratio of monoacid(s)/diacid(s) (b.1/b.2) is 0.10 to 10.0;
      - the molar ratio between organic carboxylic groups and organic hydroxyl groups (COOH/OH) present in the system is 0.30 to 0.80; and
      - the molar ratio between the compound(s) within the definition a.2 and the compound/s under definition a.1 is 0 to 0.5.
2. The mixture according to aspect 1, wherein the molar ratio of monoacid(s)/diacid(s) (b.1/b.2) is 0.2 to 8.0, preferably 0.30 to 5.0.
3. The mixture according to aspect 1 or 2, wherein the molar ratio between organic carboxylic groups and organic hydroxyl groups (COOH/OH) present in the system is 0.40 to 0.70, preferably 0.50 to 0.70
4. The mixture according to any one of the preceding aspects, wherein the molar ratio between the compound(s) within the definition a.2 and the compound/s under definition a.1 is 0 or 0.1 to 0.5, preferably 0.1 to 0.5.
5. The mixture according to any one of the preceding aspects, the amounts of the compounds a.1, a.2, b.1 and b.2 are introduced in the reaction system of Step I in amounts that result in the following molar ratios:
   A)
      - the molar ratio of monoacid(s)/diacid(s) (b.1/b.2) is 0.30 to 5.0;
      - the molar ratio between organic carboxylic groups and organic hydroxyl groups (COOH/OH) present in the system is 0.50 to 0.70; and
      - the molar ratio between the compound(s) within the definition a.2 and the compound/s under definition a.1 is 0, or is 0.1 to 0.5; or
   B)
      - the molar ratio of monoacid(s)/diacid(s) (b.1/b.2) is 1.0 to 4.0, preferably 1.5 to 3.5;
      - the molar ratio between organic carboxylic groups and organic hydroxyl groups (COOH/OH) present in the system is 0.40 to 0.70, preferably 0.50 to 0.70; and
      - the molar ratio between the compound(s) within the definition a.2 and the compound/s under definition a.1 is 0, or is 0.1 to 0.5; or
   C)
      - the molar ratio of monoacid(s)/diacid(s) (b.1/b.2) is 0.10 to 1.0, preferably 0.20 to 1.0, more preferably 0.30 to 1.0; and/or
      - the molar ratio between organic carboxylic groups and organic hydroxyl groups (COOH/OH) present in the system is 0.30 to 0.70, preferably 0.40 to 0.70, more preferably 0.50 to 0.70; and/or
      - the molar ratio between the compound(s) within the definition a.2 and the compound/s under definition a.1 is 0 or 0.1 to 0.5, preferably 0.1 to 0.5.
6. The mixture according to any one of the preceding aspects, wherein the alkanolamine(s) of formula (I) is/are selected from triethanolamine, N-methyldiethanolamine, N-methyldiisopropanolamine and triisopropanolamine, each of which is optionally alkoxylated with ethylene oxide or propylene oxide, and mixtures thereof.
7. The mixture according to any one of the preceding aspects, wherein in the dicarboxylic acid(s) of formula (III), each L is selected from ethane-1,2-diyl, 1-hydroxyethane-1,2-diyl, *cis*-ethene-1,2-diyl, *trans-*ethene-1,2-diyl, propane-1,2-diyl, propane-1,3-diyl, butane-1,4-diyl, pentane-1,5-diyl, hexane-1,6-diyl, cyclohexane-1,4-diyl, octane-1,8-diyl and 1,4-phenylenyl; preferably butane-1,4-diyl, hexane-1,6-diyl or octane-1,8-diyl.
8. The mixture according to any one of the preceding aspects, wherein the dicarboxylic acid of formula (III) is selected from succinic, malic, glutaric, adipic, sebacic, pimelic, suberic, maleic and terephthalic acid, acids obtained by thermal oligomerisation of unsaturated fatty acids, and mixtures thereof.
9. The mixture according to any one of the preceding claims, wherein the reactive derivative(s) of the dicarboxylic acid(s) of the general formula (III) are one or more selected from halide, anhydride, preferably mixed anhydride with acetic acid or cyclic anhydride.
10. The mixture according to any one of the preceding aspects, wherein the monocarboxylic acid(s) of formula (II) are synthetic fatty acids and/or are obtained from fats or oils of natural origin, and are optionally hydrogenated.
11. The mixture according to any one of the preceding aspects, wherein the monocarboxylic acid(s) of formula (II) are derived from oils of vegetal origin which are optionally hydrogenated.
12. The mixture according to any one of the preceding aspects, wherein the monocarboxylic acid(s) of formula (II) are selected from those which are obtained from tallow, palm, olive, coconut, sunflower, soya, rapeseed, grape marc and grape, each of which can be hydrogenated, partially hydrogenated, or non-hydrogenated.
13. The mixture according to any one of the preceding aspects, wherein the iodine value of the carboxylic monoacid(s) of formula (II) is preferably ≥ 5, more preferably ≥ 35, even more preferably ≥ 45, most preferably ≥ 50; and/or preferably ≤ 280, more preferably ≤ 180, even more preferably ≤ 100, most preferably ≤ 80.
14. The mixture according to any one of the preceding aspects, wherein the iodine value of the carboxylic monoacid(s) of formula (II) is 5-280, more preferably 5-180, more preferably 35-180, 35-100, more preferably 45-100, more preferably 45-80, most preferably 50-80.
15. The mixture according to any one of the preceding aspects, wherein the carboxylic monoacid(s) of formula (II) is one or more selected from caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselinic acid, linoleic acid, linolenic acid, eleostearic acid, arachic acid, gadoleic acid, behenic acid and erucic acid, and mixtures thereof which are obtained for example by pressure splitting of natural fats and oils, in the reduction of aldehydes from Roelen's oxosynthesis or dimerization of unsaturated fatty acids, stearic acids, isostearic acid, palmitic acid, myristic acid, lauric acid, capric acid, caprylic acid, 2-ethylhexanoic acid, 2-octyldodecanoic acid, capric acid, oleic acid, linoleic acid, linolenic acid, partially hydrogenated coconut fatty acid, palm fatty acid, partially hydrogenated distilled palm fatty acid, hydrogenated distilled palm fatty acid, palm kernel fatty acid, tallow fatty acid, distilled tallow fatty acid, and rapeseed fatty acid.
16. The mixture according to any one of the preceding aspects, wherein the compounds corresponding to a.1 and/or a.2 are from natural origin.
17. The mixture according to any one of the preceding aspects, wherein the compounds corresponding to a.1 and/or a.2 are from synthetic origin.
18. The mixture according to any one of the preceding aspects, wherein Step II corresponds to the formation of the addition salts of the alkanolamine esters obtained from Step I with mineral or organic acids, preferably wherein the mineral or organic acids are one or more selected from hydrochloric, sulphuric, phosphoric, citric and lactic acid.
19. The mixture according to any one of aspects 1-17, wherein Step II corresponds to the quaternisation of reaction mixtures of Step I with alkylating agent(s), preferably wherein the alkylating agents are one or more selected from methyl chloride, methyl bromide, dimethyl sulphate, diethyl sulphate and dimethyl carbonate.
20. The mixture according to any one of the preceding aspects, wherein the polyol a.2 is one or more selected from trimethylolpropane (TMP), glycerine and neopentyl glycol (NPG), each of which can be can be optionally alkoxylated, preferably ethoxylated; wherein the polyol a.2 is more preferably trimethylolpropane (TMP), or is absent.
21. The mixture according to any one of the preceding aspects, further comprising an organic solvent, preferably an alcohol, more preferably ethanol, n-propanol or isopropanol, butanols, glycol, propane or butanediol, glycerol, diglycol, propyl or butyl diglycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol methyl or ethyl ether, methoxy, ethoxy or butoxy triglycol, 1-butoxyethoxy-2-propanol, 3-methyl-3-methoxybutanol, or propylene glycol t-butyl ether.
22. The mixture according to aspect 21, wherein the content of the organic solvent is 0-30%, preferably 0-20%, more preferably 10-20% by weight.
23. The mixture according to any one of the preceding aspects, wherein the mixture is essentially water-free.
24. The mixture according to any one of the preceding aspects, wherein the mixture essentially consists of the reaction products of steps I and II, and optionally an organic solvent.
25. The mixture according to any one of the preceding aspects, wherein the mixture consists of the reaction products of steps I and II and solvent, if any, and unreacted starting materials as well as inevitable impurities from the production process, if any.
26. A softener composition, preferably a fabric-softening and/or keratin-based-fibres-softening composition, comprising the cationic surfactant mixture according to any one of the preceding aspects.
27. The composition according to aspect 26, further comprising water.
28. The composition according to aspect 26 or 27, which is optically transparent at room temperature, and preferably at temperatures of 26-40 °C, more preferably 20-50 °C, even more preferably 16-50 °C, most preferably 5-60 °C.
29. The composition according to any one of aspects 26-28, wherein:
   the water content is preferably higher than 50%, more preferably higher than 80%, most preferably higher than 85% w/w; and/or
   wherein the solid residue is lower than 50%, more preferably lower than 25%, even more preferably between 15 and 2%.
30. The composition according to any one of aspects 26-29, further comprising:
   A) a perfume, preferably wherein:
      (i) the perfume consists of one or more substance(s); and/or
      (ii) the average logP of the perfume substance(s) is from 1 to 6; and/or
      (iii) the weight ratio between the cationic surfactant mixture and the perfume is from 99:1 to 40:60, more preferably from 80:20 to 60:40, even more preferably 80:20 to 70:30; and/or
      (iv) the perfume can be partially encapsulated, preferably the perfume is encapsulated in a biodegradable microcapsule, more preferably the microcapsule is based on chitosan; and/or
   B) a non-ionic surfactant, the weight ratio between the cationic surfactant mixture and the non-ionic surfactant is from 100:0 to 70:30; and/or
   C) a thickener, wherein the weight ratio of the mixture according to aspect 1 to 24 to the thickener is from 150:1 to 10:5, preferably from 100:1 to 10:2
31. The composition according to 26-29 comprising:
   (i) the mixture of cationic surfactants as defined in any one of aspects 1 to 24, and
   (ii) optionally one or more non-ionic surfactants, in which the sum of components (i) and (ii) is from 2% to 50% by weight, wherein,
      - the content of component (i) is from 1% to 50%, preferably from 5 to 15% by weight, relative to the weight of the composition; and
      - the content of component (ii) is from 0% to 10%, preferably from 0 to 7%; more preferably from 0 to 5%, even more preferably 0 to 2%, most preferably 0 to 0.05% by weight, relative to the weight of the composition.
32. A concentrated softener composition comprising the cationic surfactant mixture according to any one of aspects 1 to 25, and having a water content lower than 50%, preferably lower than 30%, preferably lower than 5%.
33. The composition of aspect 32, which is liquid and transparent at temperature from 26-60 °C, preferably 20-40 °C, more preferably 16-60 °C, most preferably 0-80 °C.
34. The composition according to aspect 32 or 33, further comprising:
   A) a perfume, preferably wherein:
      (i) the perfume consists of one or more substance(s); and/or
      (ii) the average logP of the perfume substance(s) is from 1 to 6; and/or
      (iii) the perfume is an encapsulated perfume, preferably the perfume is encapsulated in a biodegradable microcapsule, more preferably the microcapsule is based on chitosan; and/or
   B) a non-ionic surfactant; and/or
   C) characterised in that the weight ratio of the described components are values according to the following ratios:
      P:C is a value from 0:100 to 60:40 NI:C is from 0:100 to 30:70
         - P: corresponds to the perfume, optionally microencapsulated perfume;
         - C: corresponds to the cationic surfactant mixture according to aspects 1 to 25;
         - NI: corresponds to the non-ionic surfactant.
35. A concentrated composition suitable for preparing a unit dose softener contained in a water-soluble pouch, wherein the composition:
   - comprises a mixture according to any one of aspects 1-25, and
   - is liquid and transparent at a temperature from 26-60 °C, preferably 20-60 °C, more preferably 16-80 °C, most preferably 0-80 °C,
   wherein in the system in Step I of the process for obtaining the mixture of cationic surfactants:
   - the molar ratio of monoacid(s)/diacid(s) (b.1/b.2) is 0.10 to 5.0, preferably 1.0 to 4.0, more preferably 1.5 to 3.5; and/or
   - the molar ratio between organic carboxylic groups and organic hydroxyl groups (COOH/OH) present in the system is 0.30 to 0.70; preferably 0.40 to 0.70, more preferably 0.50 to 0.70.
36. The concentrated composition according to aspect 35, which has a water content lower than 10%, preferably lower than 5%, more preferably lower than 1%, even more preferably lower than 0.1%, most preferably 0%.
37. The concentrated composition according to aspect 35 or 36, further comprising:
   A) a perfume, preferably wherein:
      (i) the perfume consists of one or more substance(s); and/or
      (ii) the average logP of the perfume substance(s) is from 1 to 6; and/or
   B) a non-ionic surfactant, which is preferably characterised in that the weight ratio of the described components are values according to the following ratios:
      P:C is a value from 0:100 to 60:40
      NI:C is 0 or a value from 0:100 to 50:50
      P corresponds to the weight content of perfume,
      C corresponds to the weight content of cationic surfactant mixture according to any of aspects 1 to 25,
      NI corresponds to the weight content of non-ionic surfactant.
38. The concentrated composition according to any one of aspects 35-37, which essentially consists, or consists of, the mixture of cationic surfactants according to any one of aspects 1-25, perfume, optionally non-ionic surfactant, and optionally an organic solvent.
39. A fabric conditioner unit dose comprising:
   - a water-soluble pouch acting as container of a liquid composition,
   - the fabric softening composition according to aspects 35-38 contained within or by the pouch, preferably wherein the unit dose is a capsule or a tablet, and preferably wherein the water soluble pouch is formed of or comprises polyvinyl alcohol.
40. A concentrated softener composition suitable for preparing a domestic softener formulation by dilution, comprising the mixture according to any one of aspects 1-25, and having a water content lower than 50%, preferably lower than 30%, preferably lower than 5%, wherein in the system in Step I of the process for obtaining the mixture of cationic surfactants:
   - the molar ratio of monoacid(s)/diacid(s) (b.1/b.2) is 0.10 to 5.0, preferably 1.0 to 4.0, more preferably 1.5 to 3.5; and/or
   - the molar ratio between organic carboxylic groups and organic hydroxyl groups (COOH/OH) present in the system is 0.30 to 0.70; preferably 0.40 to 0.70, more preferably 0.50 to 0.70.
41. The concentrated softener composition according to aspect 40, which is liquid at temperature from 26-60 °C, preferably 20-60 °C, more preferably 16-80 °C, most preferably 0-80 °C
42. The composition of any one of aspects 39-41, which is liquid and transparent at temperature from 26-60 °C, preferably 20-40 °C, more preferably 16-60 °C, most preferably 0-80 °C.
43. The composition according to any one of aspects 39-42, further comprising:
   A) a perfume, preferably wherein:
      (i) the perfume consists of one or more substance(s); and/or
      (ii) the average logP of the perfume substance(s) is from 1 to 6; and/or
      (iii) the perfume is an encapsulated perfume, preferably the perfume is encapsulated in a biodegradable microcapsule, more preferably the microcapsule is based on chitosan; and/or
   B) a non-ionic surfactant; and/or
   C) characterised in that the weight ratio of the described components are values according to the following ratios:
      P:C is a value from 0:100 to 60:40NI:C is from 0:100 to 30:70
         - P: corresponds to the perfume, optionally microencapsulated perfume;
         - C: corresponds to the cationic surfactant mixture according to aspects 1 to 24;
         - NI: corresponds to the non-ionic surfactant.
44. The composition according to aspect 30, 31, 34, 37, 38 or 43, wherein the non-ionic surfactant(s) are selected from fatty acids, linear or branched, alkoxylated or non-alkoxylated esters of fatty acids, especially those containing from 8 to 18 carbon atoms, alkoxylated or non-alkoxylated Guerbet alcohols, optionally alkoxylated glycerol and polyglycerol esters, xylitol esters, alkoxylated or non-alkoxylated sorbitan esters, esters of sugars, such as glucose, fructose, galactose, mannose, xylose, arabinose, ribose, 2-deoxyribose and sucrose, C₈₋₁₈ fatty alcohols, alkyl polyglucosides, non-ionic surfactants with amide groups derived from amines, such as glucamine, and the derivatives of methylethanolamine, diethanolamine, isopropanolamine and monoethanolamine, with linear or branched fatty acids, especially those containing from 8 to 18 carbon atoms, waxes, such as paraffins, microcrystalline waxes derived from petroleum, and synthetic waxes, and pentaerythritol esters, especially having a tallow, hydrogenated tallow, palm, behenic or oleic chain, preferably non-ionic surfactants are selected from glycerine esters that are ethoxylated, sorbitan monoesters and pentaerythritol esters, especially those having a tallow, hydrogenated tallow, palm, behenic or oleic chain.
45. The composition according to any one of aspects 26-38 or 40-44, preferably 32-38 or 40-44, characterised in having a viscosity at 20 °C of 200-50,000 cps, as measured on a Brookfield LVT viscometer with spindle 2 at 60 rpm or with spindle 4 at 12 rpm.
46. The composition according to aspect 45, wherein the composition:
   A) has a viscosity of 200 to 5,000 mPas as measured on a Brookfield LVT viscometer with spindle 4 at 12 rpm, optionally 200 to 800 mPas; or
   B) the composition has a viscosity of 200 to 800 mPas as measured on a Brookfield LVT viscometer with spindle 2 at 30 rpm.
47. Use of the mixture of cationic surfactants, the composition or the unit dose according to any one of the preceding aspects for maintaining or improving moisture wicking properties of fabrics or fibres.
48. The use of any one of the preceding aspects for maintaining or improving hydrophilic properties of cotton fabrics or fibres while providing softness.
49. The use of any one of the preceding aspects for maintaining or improving hydrophilic properties of synthetic fabrics or fibres.
50. The use of the mixture, composition or unit dose of the preceding aspects for softening fabrics and/or fibres, such as keratin-based-fibres, comprising a step of contacting the mixture, composition or unit dose with the fabrics and/or fibres.
51. The use according to aspect 48, comprising dispersing the composition of any one of aspects 32-38 or 40-46 in water, preferably tap water; or comprising introducing the unit dose of aspect 39 or the composition of any one of aspects 32-38 or 40-46 directly into a washing machine.
52. Use of the mixture of any one of aspects 1-25 for the use of any one of aspects 47-51, comprising preparing an optically transparent composition; or a process for preparing an optically transparent composition from the mixture of any one of aspects 1-25 and using it for the use of any one of aspects 47-51, wherein the molar ratio of monoacid(s)/diacid(s) (b.1/b.2) is 0.10 to 1.0, preferably 0.20 to 1.0, more preferably 0.30 to 1.0; preferably comprising combining the mixture with water.

As used herein, "clear" or "optically transparent" appearance of a mixture/composition/formulation may optionally refer to essentially complete optical transmittance, e.g., preferably ≥ 90%, more preferably ≥ 95%, even more preferably ≥ 98%, most preferably ≥ 99%. Preferably, and unless specified otherwise, the optical transmittance of such mixture/composition/formulation is measured at A = 600 nm (1 cm thickness, 20 °C).

As used herein, in cases where a ratio (e.g., molar ratio) "x/y" between compound(s) within a first definition "x" and compound(s) under a second definition "y" is 0, this means that compounds within the first definition "x" are absent or essentially absent.

As used herein, and unless specified otherwise, "stable" or "stable upon storage" may optionally refer to compositions comprising the cationic surfactant mixture according to the present invention which maintain their optical transmittance as described above immediately after their preparation and after storage. Preferably, the compositions are stable over at least 7 days at 20 °C; over at least 7 days at 5 °C; over at least 14 d at 20 °C; over at least 28 days at 20 °C; or over at least 28 days at 40 °C.

As used herein, viscosity is measured on a Brookfield LVT viscometer at 20 °C with a spindle 2 at 30 or 60 rpm (preferably: for low viscosities), or with a spindle 4 at 12 rpm (preferably: for high viscosities).

As used herein, "iodine number" (or "iodine value", IV) describes the degree of unsaturation, e.g., of a fatty acid, and can be determined according to EN 14111:2003.

As used herein, "maintaining moisture wicking properties" refers to essentially maintaining the water wicking capability of a fabric or fibre sample upon treatment with mixtures or compositions of the invention, as compared to untreated sample. Preferably, the sample is cotton or synthetic fabric; more preferably cotton fabric. Essentially maintaining the water wicking capability preferably refers to wicking results of 50%; more preferably 55% or more, even more preferably 80% or more, most preferably 95% or more; as treated and measured under the conditions described in Example 3 below after 15 minutes.

As used herein, "improving moisture wicking properties" refers to increasing the water wicking capability of a fabric or fibre sample upon treatment with mixtures or compositions of the invention, as compared to untreated sample or as compared to a sample treated with a different agent, preferably treated and measured under the conditions described in Example 3 below after 15 minutes.

### EXAMPLES

### Example 1: Synthetic procedure

### (i) Esterification:

115.1 grams (0.42 mol) of tallow fatty acid and 202.9 grams (1.39 mol) of Adipic acid were introduced in an inert atmosphere into a glass reactor. Then, 292.3g (1.96 mol) of triethanolamine, together with 65.8 grams of Trimethylolpropane (0.49 mol) were introduced, which were added with stirring. The mixture was heated for at least 4 hours at 160-180°C in order to remove water from the reaction. The final point of the reaction is monitored by an acid value assay until the value was below 2 mg KOH/g.

A yellowish liquid product from the esterification was obtained. consisting essentially of a mixture of unesterified fatty acids and adipic acid, mono-, di- and triesterified triethanolamine with fatty acids, mono-, di- and triesterified triethanolamine with adipic acid or a combination thereof, mono-, di- and triesterified trimethylolpropane with fatty acids, mono-, di- and triesterified trimethylolpropane with adipic acid or a combination thereof, together with unreacted triethanolamine and trimethylolpropane.

### (ii) Quaternisation:

611.5 grams of the product from esterification step (containing 1.94 mol of amine equivalent product) are mixed with 149.0 g of Ethanol (3.23 mol), then, 232.4 grams (1.84 mol) of dimethyl sulphate were added with stirring at a temperature of 50-90°C.

After four hours of digestion, the virtually complete absence of amine value was verified by acid/base assay. 992.9 grams of the final product was obtained.

### Calculations:

Molar ratio monocarboxylic acid / dicarboxylic acid:
0.42 mol tallow fatty acid / 1.39 mol adipic acid = 0.30

Equivalents ratio COOH / OH:
(1 eq * 0.42 mol tallow fatty acid + 2 eq * 1.39 mol adipic acid) / (3 eq * 1.96 mol triethanolamine + 3 eq * 0.49 mol trimethylolpropane) = 0.44

Molar ratio polyol / triethanolamine:
0.49 mol trimethylolpropane / 1.96 mol triethanolamine = 0.25

### Example 2: Preparation of fabric softener

Softener compositions were made by dispersing quaternary esterammonium-containing compositions into water. Aqueous dispersions shown in **Table 1** contain 10% w/w of quaternary esterammonium-containing composition.

The dispersion process consists of heating deionized water at 40 °C in a jacketed glass reactor, adding the quaternary esterammonium-containing composition of interest in the molten state (heated 5 to 10 °C above the melting point) and homogenizing the dispersion at a rate of 150 rpm during 20 min until complete incorporation. If required, a thickening polymer can be added. The aqueous dispersion is finally cooled down up to 25-30 °C, at a rate of-1.0 °C/min, maintaining the agitation at 150 rpm. If required, the fragrance is added under stirring.

### Example 3: Wicking measurement

A front load washing machine (Miele W840) was used for the washing step, configuring the machine with the white/color program, at 40°C, 900 rpm, water hardness: 20°HF. Total load: 0.5 kg of standardized 25×25cm cotton fabrics (EMPA 211) and 2 kg of cotton towels. 66.75 g of powder detergent is used.

Standardized cotton fabrics were then treated with the fabric softener using a launder-o-meter. Each cavity of the launder-o-meter had a 25x25cm standardized cotton fabric, a bath ratio of 1/10 using 20°HF water and 1.2 g/L of the fabric softener compositions. After 10 minutes of softening cycle, fabrics were rinsed with tap water and were air dried for 48 hours at 20°C and 60% RH.

Once the standardized cotton fabrics were dry, small pieces of fabric of 2.5x25 cm were cut. Each piece of fabric was placed in vertical position with 1 cm at the bottom immersed in distilled water. Water started to wet the fabric by capillarity. The height of the wetted fabric is measured at 0, 3, 6, 9, 12 and 15 minutes.

Results of wicking are expressed as the wetted height of the treated fabric divided by the wetted height of an untreated fabric in %.

**Table 1**

| **Sample** | **1** | **2** | **3** | **4** | **5** | **6** | **7** | **8** |
|---|---|---|---|---|---|---|---|---|
| **EQ ref.** | **25105** | **25094** | **25204** | **24903** | **25199** | **25267** | **25268** | **L1/90S** |
| Mono/di ratio | 0.3 | 0.6 | 1 | 1.4 | 2.5 | 7 | 10 | - |
| COOH/OH ratio | 0.6 | 0.6 | 0.6 | 0.57 | 0.6 | 0.6 | 0.6 | 0.55 |
| Polyol/TEA | 0.14 | 0 | 0.14 | 0 | 0.14 | 0.14 | 0.14 | 0 |
| Polyol | TMP | - | TMP | - | TMP | TMP | TMP | - |
| Diacid | Adipic | Adipic | Adipic | Adipic | Adipic | Adipic | Adipic | - |
| % Wicking | 110 | 99 | 104 | 101 | 94 | 69 | 57 | 52 |

## Claims

1. Use of a mixture of cationic surfactants for maintaining or improving moisture wicking properties of fabrics or fibres, wherein the mixture is obtainable from a process comprising the steps:
Step I: esterification of a) with b), and
Step II: cation formation from the reaction products of Step I,
wherein:
a) is a hydroxyl group-containing compound or a mixture of hydroxyl group-containing compounds comprising a.1 and optionally a.2, wherein:
- a.1 is an alkanolamine or a mixture of alkanolamines of the general formula (I): 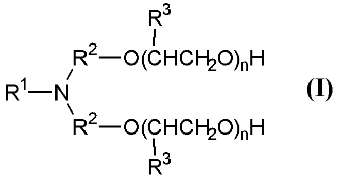 in which R¹ is selected from hydrogen, a C₁-C₆ alkyl group, and the residue 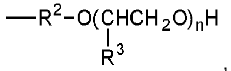 R² is a C₁-C₆ alkylene group, R³ is hydrogen or methyl, n is 0 or an integer from 1 to 20; and
- a.2 is a polyol, which can be optionally alkoxylated, and is **characterized by** a MW in the range 60 to 190 g/mol;
b) is a mixture of compounds containing one or more carboxylic groups comprising b.1 and b.2, wherein:
- b.1 is a monocarboxylic acid or a mixture of monocarboxylic acids of formula (II):
R⁶-COOH (II)
in which R⁶ is a linear or branched C₆-C₂₃ alkyl or alkenyl group; or an alkyl ester or glyceride thereof, preferably a linear or branched C₆-C₂₃ alkyl or alkenyl ester; and
- b.2 is a dicarboxylic acid or a mixture dicarboxylic acids of the general formula (III), or reactive derivative(s) thereof: 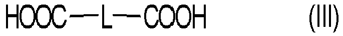
wherein L is a saturated or unsaturated, linear, branched or cyclic group having 1 to 10 carbon atoms, each of which carbon atoms is optionally substituted by a C₁-C₆ saturated or unsaturated group; and is preferably represented by (CH(R⁷))ₘ or by (C₆-C₁₀ arylene) optionally substituted by one or more R⁷, in which each R⁷ is independently a hydrogen,
OH or a C₁-C₆ saturated or unsaturated group, m is 0 or an integer from 1 to 10, wherein
for m ≥ 2, the chain (CH)ₘ optionally contains one or more double bonds and/or cyclic group(s);
wherein a.1), a.2), b.1) and b.2) are introduced in the reaction system of Step I in amounts resulting in the following molar ratios:
- the molar ratio of monoacid(s)/diacid(s) (b.1/b.2) is 0.10 to 10.0;
- the molar ratio between organic carboxylic groups and organic hydroxyl groups (COOH/OH) present in the system is 0.30 to 0.80; and
- the molar ratio between the compound(s) within the definition a.2 and the compound/s under definition a.1 is 0 to 0.5.

2. The use of the mixture according to claim 1, wherein the amounts of the compounds a.1, a.2, b.1 and b.2 are introduced in the reaction system of Step I in amounts that result in the following molar ratios:
- the molar ratio of monoacid(s)/diacid(s) (b.1/b.2) is 0.30 to 5.0;
- the molar ratio between organic carboxylic groups and organic hydroxyl groups (COOH/OH) present in the system is 0.40 to 0.70, preferably 0.50 to 0.70;
- the molar ratio between the compound(s) within the definition a.2 and the compound/s under definition a.1 is 0, or is 0.1 to 0.5.

3. The use of the mixture according to claim 1 or 2, wherein:
A) the alkanolamine(s) of formula (I) is selected from triethanolamine, N-methyldiethanolamine, N-methyldiisopropanolamine and triisopropanolamine, each of which is optionally alkoxylated with ethylene oxide or propylene oxide, and mixtures thereof; and/or
B) the dicarboxylic acid(s) of formula (III) is selected from succinic, malic, glutaric, adipic, sebacic, pimelic, suberic, maleic and terephthalic acid, acids obtained by thermal oligomerisation of unsaturated fatty acids, and mixtures thereof; and/or
C) the monocarboxylic acid(s) of formula (II) are synthetic fatty acids or are obtained from fats or oils of natural origin and are optionally hydrogenated, preferably from oils of vegetal origin which are optionally hydrogenated, preferably wherein the monocarboxylic acids of formula (II) are selected from those which are obtained from tallow, palm, olive, coconut, sunflower, soya, rapeseed, grape marc and grape, each of which can be hydrogenated, partially hydrogenated, or non-hydrogenated; and/or
D) the compounds corresponding to a.1 and/or a.2 are from natural origin.

4. The use of the mixture according to any one of the preceding claims, wherein:
A) Step II corresponds to the formation of the addition salts of the alkanolamine esters obtained from Step I with mineral or organic acids, preferably wherein the mineral or organic acids are one or more selected from hydrochloric, sulphuric, phosphoric, citric and lactic acid; or
B) Step II corresponds to the quaternisation of reaction mixtures of Step I with alkylating agent(s), preferably wherein the alkylating agents are one or more selected from methyl chloride, methyl bromide, dimethyl sulphate, diethyl sulphate and dimethyl carbonate.

5. The use of a composition comprising the mixture according to any one of the preceding claims for fabric-softening and/or keratin-based-fibres-softening.

6. The use of the according to claim 5, wherein the composition further comprises:
A) a perfume, preferably wherein:
(i) the perfume consists of one or more substance(s); and/or
(ii) the average logP of the perfume substance(s) is from 1 to 6; and/or
(iii) the weight ratio between the cationic surfactant mixture and the perfume is from 99:1 to 40:60, more preferably from 80:20 to 60:40, even more preferably 80:20 to 70:30; and/or
(iv) the perfume can be partially encapsulated, preferably the perfume is encapsulated in a biodegradable microcapsule, more preferably the microcapsule is based on chitosan; and/or
B) a non-ionic surfactant, the weight ratio between the cationic surfactant mixture and the non-ionic surfactant is from 100:0 to 70:30; and/or
C) a thickener, wherein the weight ratio of the mixture according to claim 1 to 5 to the thickener is from 150:1 to 10:5, preferably from 100:1 to 10:2

7. The use of the composition according to claim 5 or 6 further comprising water, preferably wherein the solid residue is lower than 50%, more preferably lower than 25%, even more preferably between 15 and 2%.

8. The use of the composition according to any one of claims 5 to 7 comprising:
(i) the mixture of cationic surfactants as defined in any one of claims 1 to 4, and
(ii) optionally one or more non-ionic surfactants, in which the sum of components (i), (ii) and is from 2% to 50% by weight, wherein,
- the content of component (i) is from 1% to 50%, preferably from 5 to 15% by weight, relative to the weight of the composition; and
- the content of component (ii) is from 0% to 10%, preferably from 0 to 7%; more preferably from 0 to 5%, even more preferably 0 to 2%, most preferably 0 to 0.05% by weight, relative to the weight of the composition.

9. Use of a concentrated softener composition **characterised by** the following features:
- the composition is a liquid at temperature from 26-60 °C, preferably 20-40 °C, more preferably 16-60 °C, most preferably 0-80 °C, and
- the composition comprises a mixture as defined in any one of claims 1 to 4, and
- the composition has a water content lower than 50%, preferably lower than 30%, preferably lower than 5%.

10. The use of the composition according to claim 9, wherein the composition further comprises:
A) a perfume, preferably wherein:
(i) the perfume consists of one or more substance(s); and/or
(ii) the average logP of the perfume substance(s) is from 1 to 6; and/or
(iii) the perfume is an encapsulated perfume, preferably the perfume is encapsulated in a biodegradable microcapsule, more preferably the microcapsule is based on chitosan; and/or
B) a non-ionic surfactant; and/or
C) **characterised in that** the weight ratio of the described components are values according to the following ratios:
P:C is a value from 0:100 to 60:40NI:C is from 0:100 to 30:70
P corresponds to the perfume, optionally microencapsulated perfume.
C corresponds to the cationic surfactant composition according to claims 1 to 4
NI corresponds to the non-ionic surfactant.

11. The use of the composition according to claim 9 or 10 **characterised in** having a viscosity at 20 °C of 200-50000 cps, as measured with spindle 2 at 60 rpm or with spindle 4 at 12 rpm, preferably wherein:
A) the composition has a viscosity of 200 to 5000 mPas as measured on a Brookfield LVT viscometer with spindle 4 at 12 rpm, optionally 200 to 800 mPas; or
B) the composition has a viscosity of 200 to 800 mPas as measured on a Brookfield LVT viscometer with spindle 2 at 30 rpm.

12. The use of any one of the preceding claims for maintaining or improving hydrophilic properties of cotton fabrics or fibres while providing softness.

13. The use of any one of the preceding claims for maintaining or improving hydrophilic properties of synthetic fabrics or fibres.

14. The use of the composition of any one of claims 9-11 for softening fabrics and/or fibres comprising dispersing the composition in water, or comprising introducing the composition directly into a washing machine.
